# EUROPÄISCHE PATENTSCHRIFT

(11) **EP 2 485 805 B1**
(45) Veröffentlichungstag und Bekanntmachung des Hinweises auf die Patenterteilung: **07.03.2018**
(21) Anmeldenummer: 10743054.8
(22) Anmeldetag: 13.08.2010
(51) Int. Cl.: A61K 8/46, A61K 8/49, A61Q 17/04

(54) **KOSMETISCHE ZUBEREITUNG ENTHALTEND CETEARYLSULFOSUCCINATE UND TRISBIPHENYL-TRIAZIN**
COSMETIC PREPARATION CONTAINING CETEARYL SULFOSUCCINATES AND TRISBIPHENYL TRIAZINE
PRÉPARATION COSMÉTIQUE CONTENANT DES CÉTÉARYLSULFOSUCCINATES ET DE LA TRISBIPHÉNYL-TRIAZINE

(30) Priorität: 07.10.2009 DE 102009048558
(43) Veröffentlichungstag der Anmeldung: 15.08.2012
(73) Patentinhaber: BASF SE, 67056 Ludwigshafen am Rhein (DE)
(72) Erfinder: SKUBSCH, Kerstin, 25497 Prisdorf (DE); TESCH, Mirko, 22303 Hamburg (DE); KOEHLER, Manuela, 22147 Hamburg (DE); MEYER, Christiane, 20357 Hamburg (DE)
(74) Vertreter: BASF IP Association
(86) Internationale Anmeldenummer: PCT/EP2010/004992
(87) Internationale Veröffentlichungsnummer: WO 2011/042089

(56) Entgegenhaltungen:
- DE-A1-102004 047 285
- DE-A1-102007 024 348
- DE-U1-202008 010 003
- US-A1- 2008 188 574

## Beschreibung

Die vorliegende Erfindung betrifft eine kosmetische Zubereitung enthaltend Cetearylsulfosuccinate und Trisbiphenyl-Triazin.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB-und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen. Ein Hauptnachteil von Zubereitungen, die ein oder mehrere pigmentäre organische Lichtschutzfilter, beispielsweise 2,4,6-Tris-(biphenyl)-1,3,5-triazin oder 2,2'-Methylen-bis-(6-(2H-benzotriazol-2-yl)-4-(1,1,3,3-tetramethylbutyl)-phenol) (Tinosorb M) enthalten, liegt darin, dass die Zubereitungen nicht besonders stabil sind, da es bei der Lagerung relativ schnell zur Agglomeration und Kristallbildung der organischen Filterpartikel kommt. Außerdem gibt es sehr oft bei Lagerung Öl- oder Wasserabscheidungen in der Emulsion. Dem Anwender solcher instabilen Produkte fällt dann u.a. die schlechtere Verteilbarkeit der Zubereitungen auf der Haut auf.

Es war daher die Aufgabe der vorliegenden Erfindung, die Mängel des Standes der Technik zu beseitigen und Zubereitungen mit partikulären organischen Lichtschutzfiltern zu entwickeln, die eine erhöhte Stabilität und eine geringere Neigung zur Kristallbildung aufweisen.

Überraschend gelöst werden die Aufgaben durch eine kosmetische Zubereitung enthaltend
a) Cetearylsulfosuccinate sowie
b) 2,4,6-Tris-(biphenyl)-1,3,5-triazin.

Die erfindungsgemäßen Zubereitungen sind einfach und kostengünstig mit den üblichen Herstellungsverfahren herstellbar. Die Zubereitungen weisen ein ausgesprochen angenehmes Hautgefühl, insbesondere eine nur geringe Klebrigkeit, auf. Darüber hinaus lassen sich die Zubereitungen besonders einfach auf der Haut verteilen. Gegenüber herkömmlichen partikulären organischen Lichtschutzfiltern (beispielsweise Tinosorb M) zeigt die erfindungsgemäße Stoffkombination einen höheren Lichtschutzfaktor (SPF) sowie eine deutlich geringere Gelbfärbung. Ein weiterer Vorteil besteht darin, dass man zur Herstellung stabiler Zubereitungen deutlich weniger Emulgator benötigt, als es nach dem Stand der Technik notwendig ist.

Zwar kennt der Stand der Technik die Firmenpublikation *"Eumulgin*® *Prisma, As diverse* as *the colors oft he light"* (2009) der Firma Cognis, doch konnte diese Schrift nicht den Weg zur vorliegenden Erfindung weisen, da sie nicht den erfindungsgemäßen UV-Filter offenbart.

Das erfindungsgemäße Cetearylsulfosuccinat wird erfindungsgemäß vorteilhaft als Dinatriumsalz eingesetzt. Es handelt sich bei diesem Rohstoff vorteilhaft um eine Mischung aus Dinatriumcetylsulfosuccinat: 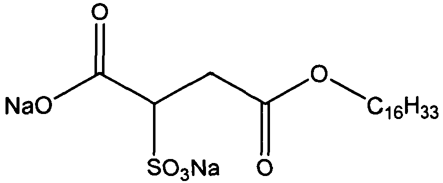 und
Dinatriumstearylsulfosuccinat:

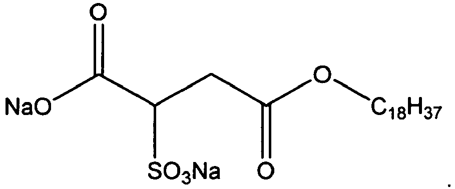

Diese Komponente ist beispielsweise bei der Firma Cognis unter dem Handelsnamen *Eumulgin*® *Prisma* erhältlich.

Es ist erfindungsgemäß vorteilhaft, wenn die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; Homosalate, Octocrylen, Ethylhexylmethoxycinnamat, Ethylhexylsalicylat, Dimethicodiethylbenzalmalonat (Polysilicone-15) und Isoamyl p-Methoxycinnamat ; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 2-Hydroxy-4-methoxybenzophenon, 2-Hydroxy-4-methoxy-4'-methylbenzophenon; 2,2'-Dihydroxy-4-methoxybenzophenon; 2-Ethylhexyl-2-hydroxybenzoat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexyl-ester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4-Bis-(4'- Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate, enthält.

Die erfindungsgemäß vorteilhaften Merocyanine werden dabei gewählt aus der Gruppe der Verbindungen

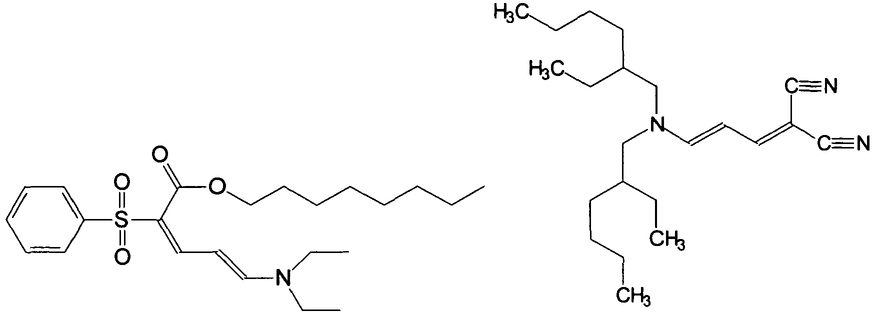

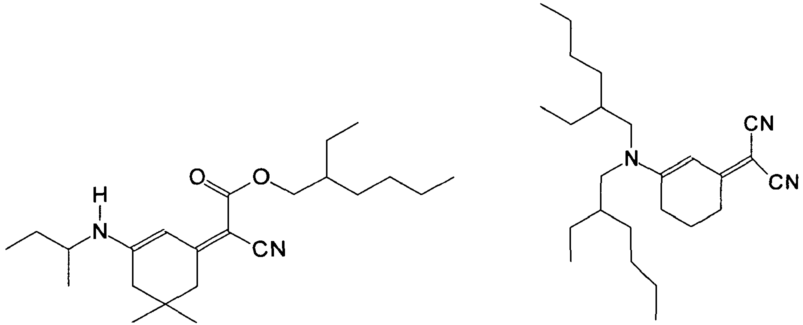

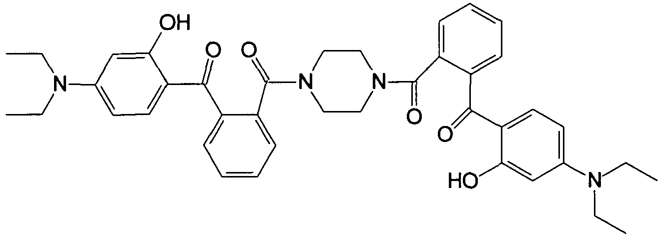

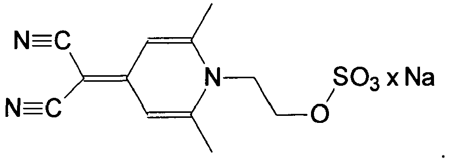

Als erfindungsgemäß vorteilhaftes Piperazinderivat kann dabei die folgende Verbindung eingesetzt werden:

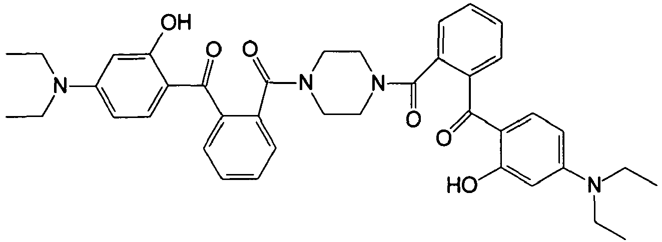

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Es ist erfindungsgemäß vorteilhaft, wenn die kosmetische Zubereitung dadurch gekennzeichnet ist, dass die Zubereitung Cetearylsulfosuccinate in einer Konzentration von 0,05 bis 5 Gewichts-%, bezogen auf das Gesamtgewicht der Zubereitung, enthält.

Es ist erfindungsgemäß bevorzugt, wenn die kosmetische Zubereitung dadurch gekennzeichnet ist, dass die Zubereitung Cetearylsulfosuccinate in einer Konzentration von 0,1 bis 2 Gewichts-%, bezogen auf das Gesamtgewicht der Zubereitung, enthält.

Es ist erfindungsgemäß vorteilhaft, wenn die Zubereitung 2,4,6-Tris-(biphenyl)-1,3,5-triazin in einer Konzentration von 0,1 bis 10 Gew.-%, bezogen auf das Gesamtgewicht der Zubereitung, enthält.

Es ist erfindungsgemäß bevorzugt, wenn die Zubereitung 2,4,6-Tris-(biphenyl)-1,3,5-triazin in einer Konzentration von 0,5 bis 5 Gew.-%, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung in Form einer Emulsion vorliegt. Dabei ist es erfindungsgemäß bevorzugt, wenn die Zubereitung in Form einer O/W-Emulsion vorliegt.

Es ist erfindungsgemäß vorteilhaft, wenn die Zubereitung frei von PEG ist.

Es ist erfindungsgemäß vorteilhaft, wenn die erfindungsgemäße Zubereitung einen oder mehrere Parfümstoffe enthält.

Die erfindungsgemäßen Parfümstoffe können beispielsweise gewählt werden aus der Gruppe der Verbindungen 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat, Vanillin, Limonen [5989-27-5], Citral, Linalool [78-70-6], alpha-Isomethylionon [1335-46-2], Geraniol [106-24-1], Citronellol [106-22-9], [24851-98-7], [18479-58-8], [54464-57-2], [80-54-6], [1222-05,5], [32388-55-9], [105-95-3], [31906-04-4], [8008-57-9], [32210-23-4], [120-57-0], [115-95-7], [101-86-0], [140-11-4], [6259-76-3] und [127-51-5].

Erfindungsgemäß besonders vorteilhafte Ausführungsformen der vorliegenden Erfindung sind dadurch gekennzeichnet, dass die Zubereitung einen oder mehrere Wirkstoffe gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, β-Alanin, Licochalcon A, Hyaluronsäure, Saponine, Dihydroxyaceton; 8-Hexadecen-1,16-dicarbonsäure, Polydocanol, enthält.

Die Wasserphase der erfindungsgemäßen Zubereitungen kann beispielsweise vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether wie Ethylenglykolmonoethyl- oder -monobutylether, Propylenglykolmonomethyl, -monoethyl- oder -monobutylether, Diethylenglykolmonomethyl-oder -monoethylether und analoge Produkte, Polymere, Schaumstabilisatoren, Elektrolyte, Verdickungsmittel, welches oder welche beispielsweise vorteilhaft gewählt werden können aus der Gruppe Siliciumdioxid, Aluminiumsilikate, Polysaccharide bzw. deren Derivate, z. B. Hyaluronsäure, Xanthangummi, Carragenanen, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON), Ultrathix P 100 von der Firma ISP (INCI: Acrylic Acid/VP Crosspolymer) sowie Aristoflex AVC (INCI: Ammonium Acryloyldimethyltaurate/VP Copolymer) und ARISTOFLEX HMB (Ammonium Acryloyldimethyltaurate/ Beheneth-25 Methacrylate Copolymer).

Die Ölphase der erfindungsgemäßen Zubereitung wird beispielsweise vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-EthylhexyNaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.
Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.
Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*HallstarAB*) und/oder Diethylhexylnaphthalat (*Hallbrite TQ oder Corapan TQ von Symrise*)*.*
Auch beliebige Abmischungen solcher Öl- und Wachskomponenten sind vorteilhaft im Sinne der vorliegenden Erfindung einzusetzen.
Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan, Polyolefine, hydrogenierte Polyisobutene, C13-16 Isoparaffin und Isohexadecan. Unter den Polyolefinen sind Polydecene die bevorzugten Substanzen.

Erfindungsgemäß bevorzugte Ausführungsformen der vorliegenden Erfindung sind dadurch gekennzeichnet, dass die Zubereitung Kohlenwasserstoffe in Form von Mineralöl enthält.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Die erfindungsgemäße Zubereitung kann ein oder mehrere der üblichen Filmbildner zur Erhöhung der Wasserfestigkeit enthalten. Es lassen sich jedoch auch erfindungsgemäß vorteilhafte Zubereitungen ohne dies Filmbildner herstellen.

Es ist erfindungsgemäß besonders vorteilhaft, wenn die Zubereitung ein oder mehrere 1,2-Alkandiole enthält. Dabei ist es erfindungsgemäß bevorzugt, eine oder mehrere Verbindungen gewählt aus der Gruppe der Verbindungen Ethylhexylglycerin, 2-Methylpropan-1,3-diol, Butylenglycol, Pentan-1,2-diol, Hexan-1,2-diol, Caprylylglycol einzusetzen.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Zur Anwendung werden die erfindungsgemäßen kosmetischen Zubereitungen in der für Kosmetika üblichen Weise auf die Haut und/oder die Haare in ausreichender Menge aufgebracht

Die erfindungsgemäße Zubereitung kann sprühbar sein.

Erfindungsgemäß vorteilhaft wird die erfindungsgemäße Zubereitung in Kunststoffflaschen aus PE, PP, PET oder Verbundstoffen aufbewahrt.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

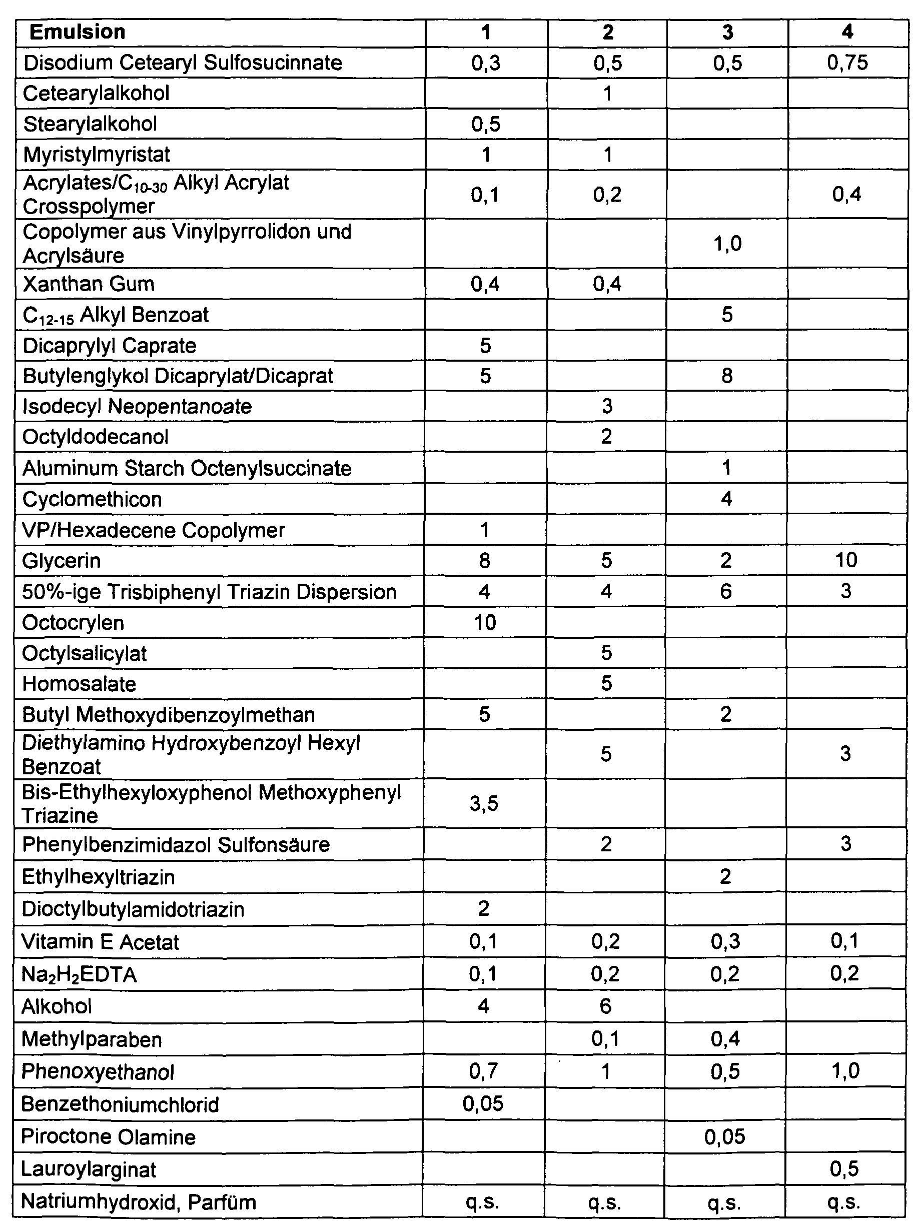

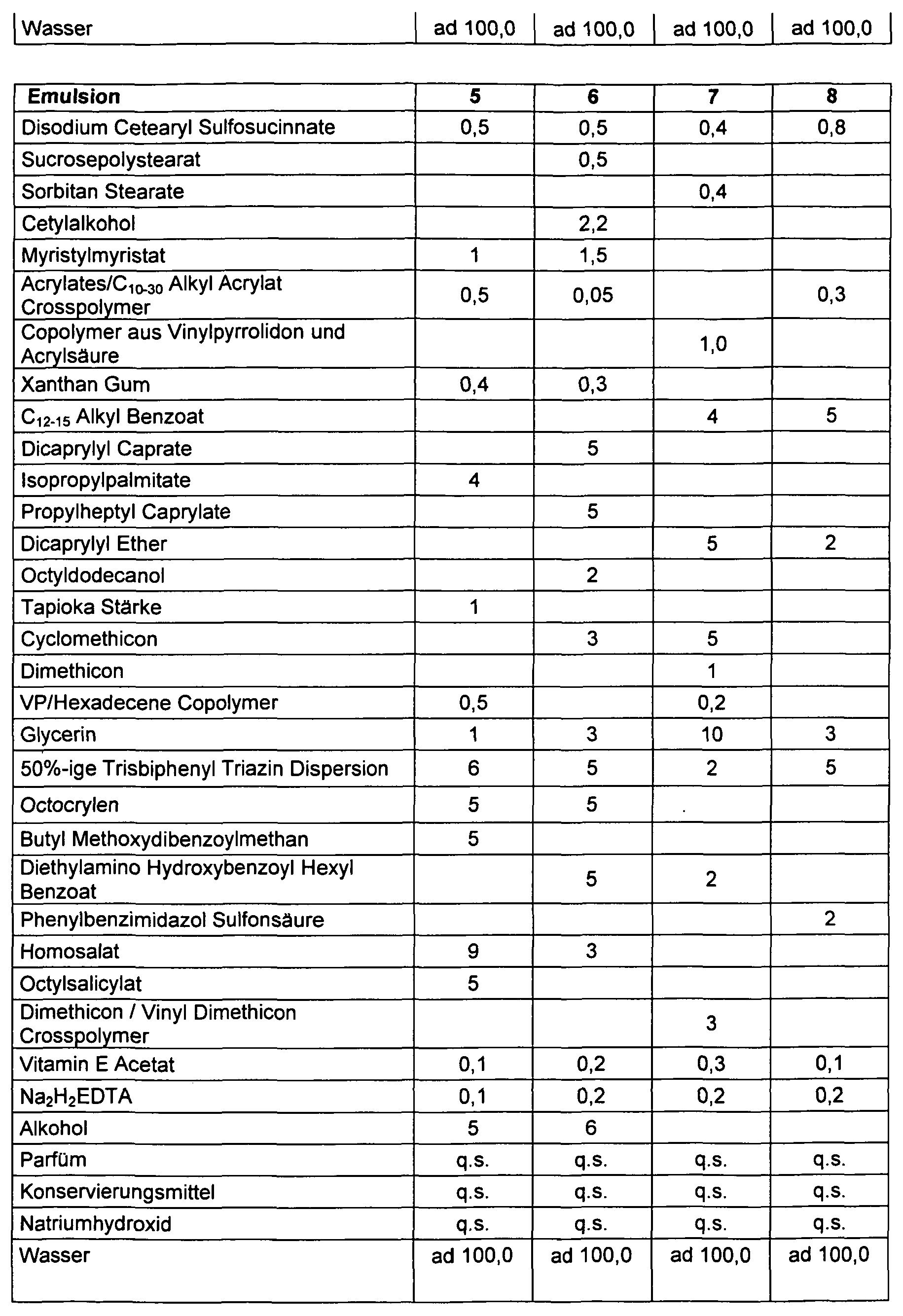

## Patentansprüche

1. Kosmetische Zubereitung enthaltend
a) Cetearylsulfosuccinate sowie
b) 2,4,6-Tris-(biphenyl)-1,3,5-triazin.

2. Kosmetische Zubereitung nach Anspruch 1, **dadurch gekennzeichnet, dass** die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; Homosalate, Octocrylen, Ethylhexylmethoxycinnamat, Ethylhexylsalicylat, Dimethicodiethylbenzalmalonat (Polysilicone-15) und Isoamyl p-Methoxycinnamat; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 2-Hydroxy-4-methoxybenzophenon, 2-Hydroxy-4-methoxy-4'-methylbenzophenon; 2,2'-Dihydroxy-4-methoxybenzophe-non; 2-Ethylhexyl-2-hydroxybenzoat; 3-(4-(2,2-bis Ethoxycarbonylvinyl)-phe-noxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoäsäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4-Bis-(4'- Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate, enthält.

3. Kosmetische Zubereitung nach einem der vorhergehenden Ansprüche, **dadurch gekennzeichnet, dass** die Zubereitung Cetearylsulfosuccinate in einer Konzentration von 0,05 bis 5 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung 2,4,6-Tris-(biphenyl)-1,3,5-triazin in einer Konzentration von 0,1 bis 10 Gew.%, bezogen auf das Gesamtgewicht der Zubereitung, enthält.

5. Kosmetische Zubereitung nach einem der vorhergehenden Ansprüche, **dadurch gekennzeichnet, dass** die Zubereitung in Form einer Emulsion vorliegt.

6. Kosmetische Zubereitung nach einem der vorhergehenden Ansprüche, **dadurch gekennzeichnet, dass** die Zubereitung in Form einer O/W-Emulsion vorliegt.

7. Kosmetische Zubereitung nach einem der vorhergehenden Ansprüche, **dadurch gekennzeichnet, dass** die Zubereitung Kohlenwasserstoffe in Form von Mineralöl enthält.

8. Kosmetische Zubereitung nach einem der vorhergehenden Ansprüche, **dadurch gekennzeichnet, dass** die Zubereitung einen oder mehrere Wirkstoffe enthält, gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, ß-Alanin, Licochalcon A, Hyaluronsäure, Saponine, Dihydroxyaceton; 8-Hexadecen-1,16-dicarbonsäure, Polydocanol.

9. Kosmetische Zubereitung nach einem der vorhergehenden Ansprüche, **dadurch gekennzeichnet, dass** die Zubereitung Cetearylsulfosuccinate in Form des Dinatriumsalzes enthält.

10. Kosmetische Zubereitung nach einem der vorhergehenden Ansprüche, **dadurch gekennzeichnet, dass** die Zubereitung ein oder mehrere 1,2-Alkandiole enthält.

## Claims

1. A cosmetic preparation comprising
a) cetearyl sulfosuccinates and
b) 2,4,6-tris(biphenyl)-1,3,5-triazine.

2. The cosmetic preparation according to claim 1, wherein the preparation comprises one or more further UV filters selected from the group of compounds comprising phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; homosalate, octocrylene, ethylhexyl methoxycinnamate, ethylhexyl salicylate, dimethicodiethylbenzalmalonate (Polysilicone-15) and isoamyl p-methoxycinnamate; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; 4-(tert-butyl)-4'-methoxydibenzoylmethane; hexyl 2-(4'-diethylamino-2'-hydoxybenzoyl)benzoate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-hydroxy-4-methoxybenzophenone, 2-hydroxy-4-methoxy-4'-methylbenzophenone; 2,2'-dihydroxy-4-methoxybenzophenone; 2-ethylhexyl 2-hydroxybenzoate; 3-(4-(2,2-bis ethoxycarbonylvinyl)phenoxy)propenyl)methoxysiloxa ne / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4-bis(4'-dineopentylaminobenzalmalonate)-6-(4"-butylaminobenzoate)-s-triazine; 4-dicyanomethylene-2,6-dimethyl-1,4-dihydropyridine-N-(ethyloxysulfate ester salt), titanium dioxide, zinc oxide, merocyanine; piperazine derivatives.

3. The cosmetic preparation according to either of the preceding claims, wherein the preparation comprises cetearyl sulfosuccinates at a concentration of 0.05 to 5% by weight, based on the total weight of the preparation.

4. The cosmetic preparation according to any of the preceding claims, wherein the preparation comprises 2,4,6-tris(biphenyl)-1,3,5-triazine at a concentration of 0.1 to 10% by weight, based on the total weight of the preparation.

5. The cosmetic preparation according to any of the preceding claims, wherein the preparation is in the form of an emulsion.

6. The cosmetic preparation according to any of the preceding claims, wherein the preparation is in the form of an O/W emulsion.

7. The cosmetic preparation according to any of the preceding claims, wherein the preparation comprises hydrocarbons in the form of mineral oil.

8. The cosmetic preparation according to any of the preceding claims, wherein the preparation comprises one or more active ingredients selected from the group of compounds comprising tocopheryl acetate, tocopherol, gylcyrrhetic acid or salts thereof, alpha-lipoic acid, folic acid, phytoene, D-biotin, coenzyme Q10, alpha-glucosylrutin, carnitine, carnosine, natural and/or synthetic isoflavonoids, glycerylglucose, creatine, creatinine, taurine, β-alanine, licochalcone A, hyaluronic acid, saponins, dihydroxyacetone; 8-hexadecene-1,16-dicarboxylic acid, polidocanol.

9. The cosmetic preparation according to any of the preceding claims, wherein the preparation comprises cetearyl sulfosuccinates in the form of the disodium salt.

10. The cosmetic preparation according to any of the preceding claims, wherein the preparation comprises one or more 1,2-alkanediols.

## Revendications

1. Préparation cosmétique contenant
a) des sulfosuccinates de cétéaryle ainsi que
b) de la 2,4,6-tris-(biphényl)-1,3,5-triazine.

2. Préparation cosmétique selon la revendication 1, **caractérisée en ce que** la préparation contient un ou plusieurs autres filtres UV choisis dans le groupe des composés : sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; homosalates, octocrylène, méthoxycinnamate d'éthylhexyle, salicylate d'éthylhexyle ; benzalmalonate de diméthicodiéthyle (Polysilicone-15) et p-méthoxycinnamate d'isoamyle ; 2,2'-méthylènebis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-(1,3,3,3-tétraméthyl-1-[(triméthylsilyl)oxy]disiloxanyl]propyl]-phénol ; 3-(4-méthylbenzylidène)camphre ; 3-benzylidènecamphre ; 4-(tert-butyl)-4'-méthoxydibenzoylméthane ; ester hexylique de l'acide 2-(4'-diéthylamino-2'-hydroxybenzoyl)-benzoïque ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; 2-hydroxy-4-méthoxybenzophénone, 2-hydroxy-4-méthoxy-4'-méthylbenzophénone ; 2,2'-dihydroxy-4-méthoxybenzophénone ; 2-hydroxybenzoate de 2-éthylhexyle ; copolymère de 3-(4-(2,2-bis(éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine présentant le n° CAS 285254-16-0 ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine INCI : Ethylhexyl Triazone) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 2,4-bis-(4'-dinéopentylaminobenzalmalonate)-6-(4"-butylaminobenzoate)-s-triazine ; sel d'ester de N-éthoxysulfate de 4-dicyanométhylène-2,6-diméthyl-1,4-dihydropyridine, dioxyde de titane, oxyde de zinc, mérocyanines, dérivés de pipérazine.

3. Préparation cosmétique selon l'une quelconque des revendications précédentes, **caractérisée en ce que** la préparation contient des sulfosuccinates de cétéaryle en une concentration de 0,05 à 5% en poids par rapport au poids total de la préparation.

4. Préparation cosmétique selon l'une quelconque des revendications précédentes, **caractérisée en ce que** la préparation contient de la 2,4,6-tris-biphényl-1,3,5-triazine en une concentration de 0,1 à 10% en poids par rapport au poids total de la préparation.

5. Préparation cosmétique selon l'une quelconque des revendications précédentes, **caractérisée en ce que** la préparation se trouve sous forme d'une émulsion.

6. Préparation cosmétique selon l'une quelconque des revendications précédentes, **caractérisée en ce que** la préparation se trouve sous forme d'une émulsion H/E.

7. Préparation selon l'une quelconque des revendications précédentes, **caractérisée en ce que** la préparation contient des hydrocarbures sous forme d'huile minérale.

8. Préparation cosmétique selon l'une quelconque des revendications précédentes, **caractérisée en ce que** la préparation contient une ou plusieurs substances actives choisies dans le groupe des composés acétate de tocophéryle, tocophérol, acide glycyrrhétinique ou ses sels, acide alpha-liponique, acide folique, phytoène, D-biotine, coenzyme Q10, alpha-glucosylrutine, carnitine, carnosine, isoflavonoïdes naturels et/ou synthétiques, glycérylglucose, créatine, créatinine, taurine, β-alanine, licochalcone A, acide hyaluronique, saponines, dihydroxyacétone ; acide 8-hexadécène-1,16-dicarboxylique, polydocanol.

9. Préparation cosmétique selon l'une quelconque des revendications précédentes, **caractérisée en ce que** la préparation contient des sulfosuccinates de cétéaryle sous forme du sel disodique.

10. Préparation cosmétique selon l'une quelconque des revendications précédentes, **caractérisée en ce que** la préparation contient un ou plusieurs 1,2-alcanediols.
